# EUROPEAN PATENT APPLICATION

(11) **EP 2 708 241 A1**
(43) Date of publication of application: **19.03.2014**
(21) Application number: 12306099.8
(22) Date of filing: 12.09.2012
(51) Int. Cl.: A61K 39/395

(54) **Recombinant Fc-fusion protein of the two Immunoglobulin domains of UNC5**

(71) Applicant: Netris Pharma, 69008 Lyon (FR)
(72) Inventor: Mehlen, Patrick, 69740 Genas (FR); Delcros, Jean-Guy, 69006 Lyon (FR); Nony, Pascale, 69008 Lyon (FR)
(74) Representative: Colombet, Alain André

(57) **Abstract**

A UNC5-fusion protein comprising the two lg-like domains of UNC5A, UNC5B, UNC5C or UNC5D and an antibody Fc-part. A pharmaceutical composition comprising such a fusion protein or a nucleic acid, vector or host cell able to produce the fusion protein. The pharmaceutical composition is useful in the treatment of cancer and especially a cancer with netrin-1 over expression.

SEQUENCE LISTING

<110>  NETRIS PHARMA

<120>  Recombinant Fc-fusion protein of the two Immunoglobulin domains
       of UNC5

<130>  BET 11L2565

<160>  11

<170>  PatentIn version 3.5

<210>  1
<211>  446
<212>  PRT
<213>  Artificial Sequence

<220>
<223>  UNC5A-fusion protein UNC5A-TRAP

<400>  1

Met Asp Phe Gly Leu Arg Leu Ile Phe Leu Val Leu Val Leu Lys Gly
1               5                   10                  15

Val Leu Cys Leu Pro His Phe Leu Val Glu Pro Glu Asp Val Tyr Ile
            20                  25                  30

Val Lys Asn Lys Pro Val Leu Leu Val Cys Lys Ala Val Pro Ala Thr
        35                  40                  45

Gln Ile Phe Phe Lys Cys Asn Gly Glu Trp Val Arg Gln Val Asp His
    50                  55                  60

Val Ile Glu Arg Ser Thr Asp Gly Ser Ser Gly Leu Pro Thr Met Glu
65                  70                  75                  80

Val Arg Ile Asn Val Ser Arg Gln Gln Val Glu Lys Val Phe Gly Leu
                85                  90                  95

Glu Glu Tyr Trp Cys Gln Cys Val Ala Trp Ser Ser Ser Gly Thr Thr
            100                 105                 110

Lys Ser Gln Lys Ala Tyr Ile Arg Ile Ala Tyr Leu Arg Lys Asn Phe
        115                 120                 125

Glu Gln Glu Pro Leu Ala Lys Glu Val Ser Leu Glu Gln Gly Ile Val
    130                 135                 140

Leu Pro Cys Arg Pro Pro Glu Gly Ile Pro Pro Ala Glu Val Glu Trp
145                 150                 155                 160

Leu Arg Asn Glu Asp Leu Val Asp Pro Ser Leu Asp Pro Asn Val Tyr
                165                 170                 175

Ile Thr Arg Glu His Ser Leu Val Val Arg Gln Ala Arg Leu Ala Asp

180                 185                 190

Thr Ala Asn Tyr Thr Cys Val Ala Lys Asn Ile Val Ala Arg Arg Arg
        195                 200                 205

Ser Ala Ser Ala Ala Val Ile Val Tyr Gly Thr Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  2

<211>  444
<212>  PRT
<213>  Artificial Sequence

<220>
<223>  UNC5B-fusion protein UNC5B-TRAP

<400>  2

Met Asp Phe Gly Leu Arg Leu Ile Phe Leu Val Leu Val Leu Lys Gly
1               5                   10                  15

Val Leu Cys Tyr Phe Leu Gln Glu Pro Gln Asp Ala Tyr Ile Val Lys
            20                  25                  30

Asn Lys Pro Val Glu Leu Arg Cys Arg Ala Phe Pro Ala Thr Gln Ile
        35                  40                  45

Tyr Phe Lys Cys Asn Gly Glu Trp Val Ser Gln Asn Asp His Val Thr
    50                  55                  60

Gln Glu Gly Leu Asp Glu Ala Thr Gly Leu Arg Val Arg Glu Val Gln
65                  70                  75                  80

Ile Glu Val Ser Arg Gln Gln Val Glu Glu Leu Phe Gly Leu Glu Asp
                85                  90                  95

Tyr Trp Cys Gln Cys Val Ala Trp Ser Ser Ala Gly Thr Thr Lys Ser
            100                 105                 110

Arg Arg Ala Tyr Val Arg Ile Ala Tyr Leu Arg Lys Asn Phe Asp Gln
        115                 120                 125

Glu Pro Leu Gly Lys Glu Val Pro Leu Asp His Glu Val Leu Leu Gln
    130                 135                 140

Cys Arg Pro Pro Glu Gly Val Pro Val Ala Glu Val Glu Trp Leu Lys
145                 150                 155                 160

Asn Glu Asp Val Ile Asp Pro Thr Gln Asp Thr Asn Phe Leu Leu Thr
                165                 170                 175

Ile Asp His Asn Leu Ile Ile Arg Gln Ala Arg Leu Ser Asp Thr Ala
            180                 185                 190

Asn Tyr Thr Cys Val Ala Lys Asn Ile Val Ala Lys Arg Arg Ser Thr
        195                 200                 205

Thr Ala Thr Val Ile Val Tyr Gly Thr Asp Lys Thr His Thr Cys Pro
    210                 215                 220

Pro Cys Pro Ala Pro Glu Leu Leu Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser His Glu Asp Pro Glu Val Lys Phe
            260                 265                 270

Asn Trp Tyr Val Asp Gly Val Glu Val His Asn Ala Lys Thr Lys Pro
        275                 280                 285

Arg Glu Glu Gln Tyr Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Ala Leu Pro Ala Pro Ile Glu Lys Thr Ile Ser Lys Ala
                325                 330                 335

Lys Gly Gln Pro Arg Glu Pro Gln Val Tyr Thr Leu Pro Pro Ser Arg
            340                 345                 350

Glu Glu Met Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Lys Leu Thr Val Asp Lys Ser Arg Trp Gln Gln
                405                 410                 415

Gly Asn Val Phe Ser Cys Ser Val Met His Glu Ala Leu His Asn His
            420                 425                 430

Tyr Thr Gln Lys Ser Leu Ser Leu Ser Pro Gly Lys
        435                 440

<210>  3
<211>  446
<212>  PRT
<213>  Artificial Sequence

<220>
<223>  UNC5C-fusion protein UNC5C-TRAP

<400>  3

Met Asp Phe Gly Leu Arg Leu Ile Phe Leu Val Leu Val Leu Lys Gly
1               5                   10                  15

Val Leu Cys Leu Pro His Phe Leu Ile Glu Pro Glu Glu Ala Tyr Ile

20                          25                          30

Val Lys Asn Lys Pro Val Asn Leu Tyr Cys Lys Ala Ser Pro Ala Thr
            35                          40                          45

Gln Ile Tyr Phe Lys Cys Asn Ser Glu Trp Val His Gln Lys Asp His
      50                          55                          60

Ile Val Asp Glu Arg Val Asp Glu Thr Ser Gly Leu Ile Val Arg Glu
65                          70                          75                          80

Val Ser Ile Glu Ile Ser Arg Gln Gln Val Glu Glu Leu Phe Gly Pro
                        85                          90                          95

Glu Asp Tyr Trp Cys Gln Cys Val Ala Trp Ser Ser Ala Gly Thr Thr
                  100                         105                         110

Lys Ser Arg Lys Ala Tyr Val Arg Ile Ala Tyr Leu Arg Lys Thr Phe
            115                         120                         125

Glu Gln Glu Pro Leu Gly Lys Glu Val Ser Leu Glu Gln Glu Val Leu
      130                         135                         140

Leu Gln Cys Arg Pro Pro Glu Gly Ile Pro Val Ala Glu Val Glu Trp
145                         150                         155                         160

Leu Lys Asn Glu Asp Ile Ile Asp Pro Val Glu Asp Arg Asn Phe Tyr
                        165                         170                         175

Ile Thr Ile Asp His Asn Leu Ile Ile Lys Gln Ala Arg Leu Ser Asp
                  180                         185                         190

Thr Ala Asn Tyr Thr Cys Val Ala Lys Asn Ile Val Ala Lys Arg Lys
            195                         200                         205

Ser Thr Thr Ala Thr Val Ile Val Tyr Gly Thr Asp Lys Thr His Thr
      210                         215                         220

Cys Pro Pro Cys Pro Ala Pro Glu Leu Leu Gly Gly Pro Ser Val Phe
225                         230                         235                         240

Leu Phe Pro Pro Lys Pro Lys Asp Thr Leu Met Ile Ser Arg Thr Pro
                        245                         250                         255

Glu Val Thr Cys Val Val Val Asp Val Ser His Glu Asp Pro Glu Val
                  260                         265                         270

Lys Phe Asn Trp Tyr Val Asp Gly Val Glu Val His Asn Ala Lys Thr
            275                         280                         285

Lys Pro Arg Glu Glu Gln Tyr Asn Ser Thr Tyr Arg Val Val Ser Val

290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  4
<211>  446
<212>  PRT
<213>  Artificial Sequence

<220>
<223>  UNC5D fusion protein UNC5D-TRAP

<400>  4

Met Asp Phe Gly Leu Arg Leu Ile Phe Leu Val Leu Val Leu Lys Gly
1               5                   10                  15

Val Leu Cys Leu Pro His Phe Ile Glu Glu Pro Asp Asp Ala Tyr Ile
            20                  25                  30

Ile Lys Ser Asn Pro Ile Ala Leu Arg Cys Lys Ala Arg Pro Ala Met
        35                  40                  45

Gln Ile Phe Phe Lys Cys Asn Gly Glu Trp Val His Gln Asn Glu His
    50                  55                  60

Val Ser Glu Glu Thr Leu Asp Glu Ser Ser Gly Leu Lys Val Arg Glu
65                  70                  75                  80

Val Phe Ile Asn Val Thr Arg Gln Gln Val Glu Asp Phe His Gly Pro
                85                  90                  95

Glu Asp Tyr Trp Cys Gln Cys Val Ala Trp Ser His Leu Gly Thr Ser
            100                 105                 110

Lys Ser Arg Lys Ala Ser Val Arg Ile Ala Tyr Leu Arg Lys Asn Phe
        115                 120                 125

Glu Gln Asp Pro Gln Gly Arg Glu Val Pro Ile Glu Gly Met Ile Val
    130                 135                 140

Leu His Cys Arg Pro Pro Glu Gly Val Pro Ala Ala Glu Val Glu Trp
145                 150                 155                 160

Leu Lys Asn Glu Glu Pro Ile Asp Ser Glu Gln Asp Glu Asn Ile Asp
                165                 170                 175

Thr Arg Ala Asp His Asn Leu Ile Ile Arg Gln Ala Arg Leu Ser Asp
            180                 185                 190

Ser Gly Asn Tyr Thr Cys Met Ala Ala Asn Ile Val Ala Lys Arg Arg
        195                 200                 205

Ser Leu Ser Ala Thr Val Val Val Tyr Gly Thr Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  5
<211>  672
<212>  DNA
<213>  Artificial Sequence

<220>
<223>  UNC5A DNA sequence

<400>  5
aagcttgccg ccaccatgga cttcggcctg cggctgatct tcctggtgct ggtgctgaag      60

ggcgtgctgt gcctgcccca cttcctggtg gaacccgagg acgtgtacat cgtgaagaac     120

aagcccgtgc tgctggtgtg caaggccgtg cccgccaccc agattttctt caagtgcaac     180

ggcgagtggg tgcgccaggt ggaccacgtg atcgagagaa gcaccgacgg cagcagcggc     240

ctgcccacaa tggaagtgcg gatcaacgtg tcccggcagc aggtggaaaa ggtgttcggc     300

ctggaagagt actggtgcca gtgcgtggcc tggtccagca gcggcaccac caagagccag     360

aaggcctaca tccggatcgc ctacctgcgg aagaacttcg agcaggaacc cctggccaaa     420

gaggtgtccc tggaacaggg catcgtgctg ccctgcagac cccctgaggg cattccccct     480

gccgaggtgg aatggctgcg gaacgaggac ctggtggacc ccagcctgga ccccaatgtg     540

tacatcaccc gcgagcacag cctggtggtg agacaggccc gcctggccga caccgccaac     600

tacacctgtg tggccaagaa catcgtggcc agacggcgct ctgcctctgc cgccgtgatc     660

gtgtacggta cc                                                         672

<210>  6
<211>  666
<212>  DNA
<213>  Artificial Sequence

<220>
<223>  UNC5B DNA sequence

<400>  6

aagcttgccg ccaccatgga cttcggcctg cggctgatct tcctggtgct ggtgctgaag 60

ggcgtgctgt gctactttct gcaagaaccc caggacgcct acatcgtgaa gaacaagccc 120

gtggaactgc ggtgccgggc cttccctgcc acccaaatct acttcaagtg caacggcgag 180

tgggtgtccc agaacgacca cgtgacccag gaaggcctgg acgaggccac aggcctgaga 240

gtgcgcgagg tgcagatcga ggtgtcccgg cagcaggtgg aagaactgtt cggcctggaa 300

gattactggt gccagtgcgt ggcctggtct agcgccggca ccaccaagag cagacgggcc 360

tacgtgcgga tcgcctacct gcggaagaac ttcgaccagg aacccctggg caaagaggtg 420

cccctggacc acgaggtgct gctgcagtgc agacctcctg agggcgtgcc cgtggccgag 480

gtggaatggc tgaagaacga ggacgtgatc gaccctaccc aggataccaa cttcctgctg 540

accatcgacc acaacctgat catccggcag gcccggctga gcgacaccgc caattacacc 600

tgtgtggcca agaacatcgt ggccaagcgg cggagcacca ccgccaccgt gatcgtgtac 660

ggtacc 666

<210> 7
<211> 672
<212> DNA
<213> Artificial Sequence

<220>
<223> UNC5C DNA sequence

<400> 7
aagcttgccg ccaccatgga cttcggcctg cggctgatct tcctggtgct ggtgctgaag 60

ggcgtgctgt gcctgcccca cttcctgatc gagcccgaag aggcctacat cgtgaagaac 120

aagcccgtga acctgtactg caaggccagc cccgccaccc aaatctactt caagtgcaac 180

agcgagtggg tgcaccagaa agaccacatc gtggacgagc gggtggacga gacaagcggc 240

ctgatcgtgc gcgaggtgtc catcgagatc agccggcagc aggtggaaga actgttcggc 300

cccgaggact actggtgcca gtgcgtggcc tggtctagcg ccggcaccac caagagccgg 360

aaggcctacg tgcggatcgc ctacctgaga aagaccttcg agcaggaacc cctgggcaaa 420

gaggtgtccc tggaacaaga agtgctgctg cagtgcagac cccccgaggg aatccccgtg 480

gccgaggtgg aatggctgaa gaacgaggac atcatcgacc ccgtggaaga tcggaacttc 540

tacatcacca tcgaccacaa cctgatcatc aagcaggccc ggctgagcga caccgccaac 600

tacacctgtg tggccaagaa catcgtggcc aagcggaagt ccaccaccgc caccgtgatc 660

gtgtacggta cc 672

<210> 8
<211> 672
<212> DNA
<213> Artificial Sequence

<220>
<223> UNC5D DNA sequence

<400> 8

aagcttgccg ccaccatgga cttcggcctg cggctgatct tcctggtgct ggtgctgaag 60

ggcgtgctgt gcctgcccca cttcatcgaa gagcccgacg acgcctacat catcaagagc 120

aaccccatcg ccctgcgctg caaggcccgc cccgccatgc agattttctt caagtgcaac 180

ggcgagtggg tgcaccagaa cgagcacgtg agcgaggaga ccctggacga gagcagcggc 240

ctgaaggtgc gggaagtgtt catcaacgtg acacggcagc aggtggaaga cttccacggc 300

cccgaagact actggtgcca gtgcgtggcc tggtcccacc tgggcaccag caagagccgg 360

aaggccagcg tgcggatcgc ctacctgcgg aagaacttcg agcaggaccc ccagggccgg 420

gaggtgccca tcgagggcat gatcgtgctg cactgcagac cccctgaggg cgtgcccgcc 480

gccgaggtgg aatggctgaa gaacgaggag cccattgaca gcgagcagga cgagaatatt 540

gacacccgcg ccgaccacaa tctgattatt agacaggccc gcctgagcga cagcggcaac 600

tacacctgta tggccgccaa catcgtggcc aagcggcgct ctctgtctgc caccgtggtg 660

gtgtacggta cc 672

<210> 9
<211> 699
<212> DNA
<213> Artificial Sequence

<220>
<223> Human IgG1 Fc coding sequence

<400> 9
ggtaccgaca agacccacac ctgtccccca tgccctgccc ctgaactgct gggaggcccc 60

agcgtgttcc tgttcccccc aaagcccaag gacaccctga tgatcagccg gacccccgaa 120

gtgacctgcg tggtggtgga cgtgtcccac gaggaccctg aagtgaagtt caattggtac 180

gtggacggcg tggaagtgca caacgccaag accaagccca gagaggaaca gtacaacagc 240

acctaccggg tggtgtccgt gctgaccgtg ctgcaccagg actggctgaa cggcaaagag 300

tacaagtgca aagtctccaa caaggccctg cctgccccca tcgagaaaac catcagcaag 360

gccaagggac agccccgcga gcctcaggtg tacacactgc cccccagccg ggaagagatg 420

accaagaacc aggtgtccct gacctgcctg gtcaagggct tttaccccag cgatatcgcc 480

gtggaatggg agagcaacgg ccagcccgag aacaattaca agaccacccc ccctgtgctg 540

gacagcgacg gctcattctt cctgtacagc aagctgaccg tggacaagag ccggtggcag 600

cagggcaacg tgttcagctg cagcgtgatg cacgaggccc tgcacaacca ctacacccag 660

aagtccctga gcctgagccc cggcaagtaa taatctaga 699

<210> 10
<211> 25
<212> DNA
<213> Artificial Sequence

<220>
<223> Forward primer

<400> 10

aaaagtactg caagaaggac tatgc 25

<210> 11
<211> 21
<212> DNA
<213> Artificial Sequence

<220>
<223> Reverse primer

<400> 11
ccctgcttat acacggagat g 21

## Description

The present invention relates to a UNC5-fusion protein comprising the two Immunoglobulin domains of the ectodomain of a human receptor UNC5 and an antibody Fc part, nucleic acid molecules encoding the same and its production and use for the treatment of cancer.

Netrin-1 is a member of the netrin family and displays an axon navigation cue, both, in an attractive and repulsive context and plays a major role in the development of the nervous system (Serafini, 1996, Cell 87: 1001-1014). The main receptors for netrin-1 are DCC (Deleted in Colorectal Cancer) and UNC5 (UNC5A, UNC5B, UNC5C, UNC5D in human, UNC5H1, UNC5H2, UNC5H3, UNC5H4 in mice), which all belong to the dependence receptor family (Keino-Masu, 1996, Cell 87: 175-185 ; Ackermann, 1997, Nature 386: 838-842; Hong, 1999, Cell 97: 927-941; Mehlen, 1998, Nature 395: 801-804). Dependence receptors share the ability to induce apoptosis in the absence of their respective ligands, whereby this ability is blocked upon binding of the respective ligand (Mehlen, 2004, Cell Mol Life Sci 61: 1854-1866; Bredesen, 2005, Cell Death Differ 12: 1031-1043).

In various human cancers, reduction or loss of expression of DCC and, thus, reduction or loss of DCC-induced apoptosis has been observed (Kinzler, 1996, Proc Natl Acad Sci 100: 4173-4178). Furthermore, it has been observed that also UNC5 genes are downregulated in most colorectal tumors, indicating that the loss of dependence receptor UNC5 represents a selective advantage for tumor cells (Bernet, 2007, Gastroenterology 133: 180-1848; Shin, 2007, Gastroenterology 133: 1849-1857). However, not only down-regulation of the dependence receptor DCC and UNC5 enhances survival of various tumor cells, autocrine expression of their ligand netrin-1 has been observed. Particularly, it has been shown that the majority of breast tumors, i.e. metastatic breast cancers, exhibit increased expression of netrin-1 (Fitamant, 2008, Proc Natl Acad Sci 105: 4850-4855). Up to now, it has been demonstrated that the two Immunoglobulin (lg) subdomains of the extracellular part of UNC5 are responsible for binding of netrin-1, but it is not clear whether both domains are necessary to full netrin-1 binding (Geisbrecht, 2003, J .Biol. Chem. 278 : 32561-32568 ; Kruger, 2004, J. Neur. 24 : 10826-10834).

As has been shown previously, neutralization of netrin-1 by the ectodomain of DCC or part of this ectodomain (acting as netrin-1 decoy protein) can induce apoptosis in tumor cells expressing dependence receptors DCC and/or UNC5 (EP-A1-1 989 546). This ectodomain or part of this ectodomain is capable to reduce metastasis of breast cancer cells into the lung (Fitamant et al. 2008). Furthermore, this ectodomain or part of this ectodomain has been also demonstrated to increase the cell death percentage of non-small cell lung cancer cells and neuroblastoma cells expressing high levels of netrin-1 (Delloye-Bourgeois, 2009, J Natl Cancer Inst 101 : 237-247 ; Delloye-Bourgeois, 2009, JEM 206 : 833-847). WO2012025618 discloses DCC-fusion proteins having improved DCC decoy.

There remains a need for molecules that may interfere in an efficient way with the netrin-1 pathway, in order to favour apoptosis of cancer cells, in particular of cancer cells that over-express netrin-1. To this aim, the inventors have postulated that the molecule must have sufficient affinity for netrin-1 and stability in vivo in view of proposing a netrin-1 decoy molecule. These goals have been attained by the fusion proteins as disclosed and provided herein.

The present invention thus relates to an UNC5-fusion protein comprising the two lg-like domains of UNC5 and an antibody Fc part, which fusion protein is called herein UNC5-TRAP.

In an embodiment, UNC5 is UNC5A. The fusion protein is named herein UNC5A-TRAP. In another embodiment, UNC5 is UNC5B. The fusion protein is named herein UNC5B-TRAP. In still another embodiment, UNC5 is UNC5C. The fusion protein is named herein UNC5C-TRAP. In still another embodiment, UNC5 is UNC5D. The fusion protein is named herein UNC5D-TRAP.

In a preferred embodiment, the Fc part is the Fc or part thereof of a human IgG. The human IgG may be namely IgG1, IgG2A, IgG2B, IgG3. In a preferred embodiment, the IgG is IgG1.

In an embodiment, the fusion protein is single chain, which means that the protein is made of a UNC5 fragment comprising or constituted of the two lg-like domains of UNC5 and of a peptidic or protein sequence improving the pharmaceutical parameters of the compound.

In another and preferred embodiment, the fusion protein is double chain, which means that the fusion protein is made of two chains each comprising or constituted of respectively the two lg-like domains of UNC5 and of an antibody Fc part, wherein both chains are linked together, preferably by one or more, e.g. two, disulfide bonds.

The fusion proteins according to the invention, especially the UNC5A-Fc, UNC5B-Fc, UNC5C-Fc, UNC5D-Fc fusion proteins, have a binding affinity to netrin-1 and may act as a new compound acting as netrin-1 decoy protein and neutralizing netrin-1 function. These fusion proteins are able to inhibit interaction of netrin-1 and the netrin-1 receptors such as DCC and UNC5 (UNC5A, UNC5B, UNC5C and UNC5D). The fusion leads to the improvement of the pharmacologic properties of the compound compared to the two Ig-like domains of the UNC5 alone. The UNC5-fusion proteins of the present invention can be produced with high efficiency in HEK 293 cells in transient expression. UNC5A production in HEK 293 cells may raise values over 300mg/l.

Generally, the UNC5-fusion proteins provided in the present invention are binding molecules comprising the two Ig-like domains of UNC5A, UNC5B, UNC5C or UNC5D, and an Fc-part of human IgG1.

In an embodiment, the UNC5-fusion proteins (UNC5A, UNC5B, UNC5C or UNC5D) comprise a signal sequence, such as the kappa2 signal sequence. In an embodiment, the mature UNC5-fusion proteins do not comprise the signal sequence.

In an embodiment, the Fc sequence is at the C-terminal end of the UNC5 sequence.

In an embodiment, a linker is present between the Fc sequence and the UNC5 sequence. Preferably, this linker is placed between the C-terminal end of the UNC5 sequence and the Fc sequence. Such a linker may be the amino acids GT.

In an embodiment, the UNC5A protein in UNC5A-fusion comprises or consists of the amino acids 20 to 217 of SEQ ID NO: 1. This fusion protein may further comprise the IgG1 Fc comprising or consisting of amino acids 220 to 446 of SEQ ID NO: 1. This Fc is fused to the UNC5A protein, for example through a linker, such as GT. In an embodiment, the present invention relates to an UNC5A-fusion of UNC5A protein comprising or consisting of the amino acid sequence of SEQ ID NO: 1: Kappa2 signal peptide sequence: aas 1 to 19; lg-like domains of UNC5A: aas 20 to 217; Linker: aas 218-219; Human IgG1 Fc: aas 220 to 446. In an embodiment, the mature fusion protein does not comprise the Kappa2 signal peptide sequence. In a preferred embodiment, the fusion protein is double chain.

The present invention also encompasses variant sequences having a percentage of identity which is equal or more than 90%, preferably than 96, 95, 94, 93, 92 or 91 %, on the whole length of the 20-217 amino acid sequence, or of amino acids 20-446 of SEQ ID NO: 1. Amino acid substitutions may for example occur at one or several of positions 9, 72, 74, 87, 144, 164, 170, 193 and/or 210 on the whole length of the 20-217 amino acid sequence, or of SEQ ID NO: 1.

In another embodiment, the UNC5B protein in UNC5B-fusion comprises or consists of the amino acids 20 to 215 of SEQ ID NO: 2. This fusion protein may further comprise the IgG1 Fc comprising or consisting of amino acids 218 to 444 of SEQ ID NO: 2. This Fc is fused to the UNC5A protein, for example through a linker, such as GT. In an embodiment, the present invention relates to an UNC5B-fusion of UNC5B protein comprising or consisting of the amino acid sequence of SEQ ID NO: 2: Kappa2 signal peptide sequence: aas 1 to 19; lg-like domains of UNC5B: aas 20 to 215; Linker: aas 216-217; Human IgG1 Fc: aas 218 to 444. In an embodiment, the mature fusion protein does not comprise the Kappa2 signal peptide sequence. In a preferred embodiment, the fusion protein is double chain.

The present invention encompasses variant sequences having a percentage of identity which is equal or more than 90%, preferably than 96, 95, 94, 93, 92 or 91%, on the whole length of the 20-215 amino acid sequence, or of amino acids 20-444 of SEQ ID NO: 2. Amino acid substitutions may for example occur at one or several of positions 29, 74, 100, 109, 113, 146, 149, 155, 172, 184, 189, 201, 213 and/or 214 on the whole length of the 20-215 amino acid sequence, or of SEQ ID NO: 2.

In still another embodiment, the UNC5C protein in UNC5C-fusion comprises or consists of the amino acids 20 to 217 of SEQ ID NO: 3. This fusion protein may further comprise the IgG1 Fc comprising or consisting of amino acids 220 to 446 of SEQ ID NO: 3. This Fc is fused to the UNC5A protein, for example through a linker, such as GT. In an embodiment, the present invention relates to an UNC5C-fusion of UNC5C protein comprising or consisting of the amino acid sequence of SEQ ID NO: 3: Kappa2 signal peptide sequence: aas 1 to 19; lg-like domains of UNC5C: aas 20 to 217; Linker: aas 218-219; Human IgG1 Fc: aas 220 to 446. In an embodiment, the mature fusion protein does not comprise the Kappa2 signal peptide sequence. In a preferred embodiment, the fusion protein is double chain.

The present invention encompasses variant sequences having a percentage of identity which is equal or more than 90%, preferably than 96, 95, 94, 93, 92 or 91%, on the whole length of the 20-217 amino acid sequence, or of amino acids 20-446 of SEQ ID NO: 3. Amino acid substitutions may for example occur at one or several of positions 33, 66, 109, 129, 136, 178, 189 and/or 211 on the whole length of the 20-217 amino acid sequence, or of SEQ ID NO: 3.

In still another embodiment, the UNC5D protein in UNC5D-fusion comprises or consists of the amino acids 20 to 217 of SEQ ID NO: 4. This fusion protein may further comprise the IgG1 Fc comprising or consisting of amino acids 220 to 446 of SEQ ID NO: 4. This Fc is fused to the UNC5A protein, for example through a linker, such as GT. In an embodiment, the present invention relates to an UNC5D-fusion of UNC5D protein comprising or consisting of the amino acid sequence of SEQ ID NO: 4: Kappa2 signal peptide sequence: aas 1 to 19; lg-like domains of UNC5D: aas 20 to 217; Linker: aas 218-219; Human IgG1 Fc: aas 220 to 446. In an embodiment, the mature fusion protein does not comprise the Kappa2 signal peptide sequence. In a preferred embodiment, the fusion protein is double chain.

The present invention encompasses variant sequences having a percentage of identity which is equal or more than 90%, preferably than 96, 95, 94, 93, 92 or 91%, on the whole length of the 20-217 amino acid sequence, or of amino acids 20-446 of SEQ ID NO: 4. Amino acid substitutions may for example occur at one or several of positions 38, 79, 80, 115, 131, 178, 186, 201 and/or 212 on the whole length of the 20-217 amino acid sequence, or of SEQ ID NO: 4.

In an embodiment, in the UNC5-fusion proteins, the human IgG1 Fc sequence comprises or consists of the amino acids 220-446 of SEQ ID NO: 1, 3 or 4, or 218-444 of SEQ ID NO: 2.

The fusion proteins of the invention and the pharmaceutical compositions containing at least one of them may be used as a medicament, in particular a medicament to treat a cancer with over-expression of netrin-1. This cancer may also express DCC and/or UNC5A and/or UNC5B and/or UNC5C and/or UNC5D.

Hence, the present invention relates to UNC5-fusion proteins (UNC5A-Fc, UNC5B-Fc, UNC5C-Fc, UNC5D-Fc) as described and provided herein for use as a medicament. Particularly, the present invention relates to UNC5-fusion proteins as described and provided herein for use as a medicament for treating cancer. Preferably, the cancer to be treated is characterized in that the cancer cells over-express netrin-1. This cancer may also express the dependence receptors DCC and/or UNC5A, B, C, D on the cell surface.

Some embodiments of cancers include metastatic breast cancer, non-small cell lung cancer, aggressive neuroblastoma, pancreatic adenocarcinoma, primary melanoma (n=7), melanoma metastasis (n=6), ovarian cancers, glioblastoma, acute myeloid leukemia, chronic lymphocytic leukemia, aggressive B-cell lymphoma, sarcoma, renal adenocarcinoma, head and neck cancers, Testicular cancers (e.g. embryonal carcinoma, teratoma, yolk sac tumors), kidney cancers, stomach cancers, uterus cancers. In an embodiment, the fusion proteins of the invention and the pharmaceutical compositions containing at least one of them may be used to treat such a cancer with over-expression of netrin-1. In an embodiment, the fusion proteins of the invention and the pharmaceutical compositions containing at least one of them may be used to treat such a cancer in a patient displaying this cancer with a form that over-expresses netrin-1. These cancers may also expresses DCC and/or UNC5A and/or UNC5B and/or UNC5C and/or UNC5D.

Methods of determining whether a given cell expresses dependence receptors DCC and/or UNC5 on the surface or shows significant up-regulation of netrin-1 gene expression are well known in the art and comprise, but are not limited to, IHC (Immunohistochemistry) of FACS (Fluorescence activated cell sorting), quantitative PCR (e.g. with hexamer primed cDNA) or alternatively Western Blot paired with chromogenic dye-based protein detection techniques (such as silver or coomassie blue staining) or fluorescence- and luminescence-based detection methods for proteins in solutions and on gels, blots and microarrays, such as immunostaining, as well as immunoprecipitation, ELISA, microarrays, and mass spectrometry. In the context of the present invention, examples for cancers to be treated by UNC5-fusion protein of the present invention are listed herein including refractory versions of any of the mentioned cancers. Netrin-1 overexpression may thus be measured through RT-PCR using suitable primers as those disclosed and provided herein, with respect to normal tissue or to a similar cancer which does not overexpress netrin-1.

Accordingly, the present invention relates to a UNC5A-fusion protein as provided herein, in particular comprising or consisting the amino acids 20-217 and 220-446 of SEQ ID NO: 1 fused together, or the sequence SEQ ID NO:1, preferably amino acids 20-446 of SEQ ID NO:1, for use in treating cancer. The present invention relates to a UNC5B-fusion protein as provided herein, in particular comprising or consisting of the amino acids 20-215 and 218-444 of SEQ ID NO: 2 fused together, or the sequence SEQ ID NO: 2, preferably amino acids 20-444 of SEQ ID NO:2, for use in treating cancer. The present invention relates to a UNC5C-fusion protein as provided herein, in particular comprising or consisting of the amino acids 20-217 and 220-446 of SEQ ID NO: 3 fused together, or the sequence SEQ ID NO:3, preferably amino acids 20-446 of SEQ ID NO:3, for use in treating cancer. The present invention relates to a UNC5D-fusion protein as provided herein, in particular comprising or consisting of the amino acids 20-217 and 220-446 of SEQ ID NO: 4 fused together, or the sequence SEQ ID NO:4, preferably amino acids 20-446 of SEQ ID NO:4, for use in treating cancer. The cancer may be one of those as described herein.

The present invention also relates to a nucleic acid molecule encoding an UNC5 fusion protein as described and provided herein. Accordingly, the present invention relates to a nucleic acid molecule encoding the amino acids 20-217 and 220-446 of SEQ ID NO: 1 fused together, or the sequence SEQ ID NO:1. The invention also relates to a nucleic acid molecule encoding the amino acids 20-215 and 218-444 of SEQ ID NO: 2 fused together, or the sequence SEQ ID NO: 2. The invention also relates to a nucleic acid molecule encoding the amino acids 20-217 and 220-446 of SEQ ID NO: 3 fused together, or the sequence SEQ ID NO:3. The invention also relates to a nucleic acid molecule the amino acids 20-217 and 220-446 of SEQ ID NO: 4 fused together, or the sequence SEQ ID NO:4.

The present invention thus relates also to the following nucleic acid molecules:
- SEQ ID NO: 5 encoding an UNC5A protein; nt (nucleotides); nt 1-6 Hindlll restriction site, nt 7-15 kozak sequence, nt 16-72 kappa2 signal sequence, nt 73-666 UNC5A coding sequence, nt 667-672 Xpnl restriction site;
- SEQ ID NO: 6 encoding an UNC5B protein; nt (nucleotides); nt 1-6 Hindlll restriction site, nt 7-15 kozak sequence, nt 16-72 kappa2 signal sequence, nt 73-660 UNC5B coding sequence, nt 661-666 Xpnl restriction site;
- SEQ ID NO: 7 encoding an UNC5C protein; nt (nucleotides); nt 1-6 Hindlll restriction site, nt 7-15 kozak sequence, nt 16-72 kappa2 signal sequence, nt 73-666 UNC5C coding sequence, nt 667-672 Xpnl restriction site;
- SEQ ID NO: 8, encoding an UNC5D protein; nt (nucleotides); nt 1-6 Hindlll restriction site, nt 7-15 kozak sequence, nt 16-72 kappa2 signal sequence, nt 73-666 UNC5C coding sequence, nt 667-672 Xpnl restriction site;
- SEQ ID NO: 9 encoding a human IgG1 Fc (hinge + CH2 + CH3 DNA sequence, nt 7-693), with Kpnl restriction site at positions 1-6 nt and Xbal restriction site at position 694-699.

Other objects of the invention are hence:
- A polynucleotide (or nucleic acid or nucleotide sequence) coding for a fusion protein UNC5A-TRAP, UNC5B-TRAP, UNC5C-TRAP or UNC5D-TRAP as described and disclosed herein;
- A polynucleotide coding for a fusion protein UNC5A-TRAP, UNC5B-TRAP, UNC5C-TRAP or UNC5D-TRAP, which fusion protein comprises amino acids 20 to 217 of SEQ ID NO: 1, amino acids 20 to 215 of SEQ ID NO: 2, amino acids 20 to 217 of SEQ ID NO: 3, and amino acids 20 to 217 of SEQ ID NO: 4, respectively; encompasses the variations of amino acid sequence for the UNC5 and the fragments as disclosed herein
- A polynucleotide coding for the fusion protein of SEQ ID NO: 1, preferably amino acids 20-446 of SEQ ID NO:1, SEQ ID NO: 2, preferably amino acids 20-444 of SEQ ID NO:2, SEQ ID NO: 3, preferably amino acids 20-446 of SEQ ID NO:3, or SEQ ID NO: 4, preferably amino acids 20-446 of SEQ ID NO:4; encompasses the variations of amino acid sequence for the UNC5 and the fragments as disclosed herein;
- A polynucleotide sequence comprising or consisting of SEQ ID NO: 5 fused to SEQ ID NO: 9, in particular through a restriction site, such as *Kpnl* restriction site; encompasses variants due to degeneracy of the code;
- A polynucleotide sequence comprising or consisting of SEQ ID NO: 6 fused to SEQ ID NO: 9, in particular through a restriction site, such as *Kpnl* restriction site; encompasses variants due to degeneracy of the code;
- A polynucleotide sequence comprising or consisting of SEQ ID NO: 7 fused to SEQ ID NO: 9, in particular through a restriction site, such as *Kpnl* restriction site; encompasses variants due to degeneracy of the code.
- A polynucleotide sequence comprising or consisting of SEQ ID NO: 8 fused to SEQ ID NO: 9, in particular through a restriction site, such as *Kpnl* restriction site; encompasses variants due to degeneracy of the code.

The nucleic acid molecules of the present invention may be DNA molecules or RNA molecules. They may also be nucleic acid analogues, such as oligonucleotide thiophosphates, substituted ribo-oligonucleotides, LNA (Locked nucleic acid) molecules, PNA (Peptide nucleic acid) molecules, GNA (glycol nucleic acid) molecules, TNA (threose nucleic acid) molecules, morpholino polynucleotides, or antagomir (cholesterol-conjugated) nucleic acid molecules or any modification thereof as known in the art (see., e.g. US 5,525,711, US 4,711,955, US 5,792,608 or EP 302 175 for examples of modifications). Nucleic acid molecules in context of the present invention may be naturally occurring nucleic acid residues or artificially produced nucleic acid residues. Examples for nucleic acid residues are adenine (A), guanine (G), cytosine (C), thymine (T), uracil (U), xanthine (X), and hypoxanthine (HX). In context of the present invention, thymine (T) and uracil (U) may be used interchangeably depending on the respective type of nucleic acid molecule. For example, as the skilled person is well aware of, a thymine (T) as part of a DNA corresponds to an uracil (U) as part of the corresponding transcribed mRNA. The nucleic acid molecule of the present invention may be single- or double-stranded, linear or circular, natural or synthetic, and, if not indicated otherwise, without any size limitation. The nucleic acid molecule may also comprise a promoter as further detailed herein below. The promoter may be homologous or heterologous. In a particular embodiment, the nucleic acid molecule provided herein is under the control of this promoter.

Generally, as used herein, a polynucleotide comprising the nucleic acid sequence of a sequence provided herein may also be a polynucleotide consisting of said nucleic acid sequence.

Furthermore, in accordance with the present invention, the nucleic acid molecule of the present invention may be cloned into a vector. The term "vector" as used herein particularly refers to plasmids, cosmids, viruses, bacteriophages and other vectors commonly used in genetic engineering. In a preferred embodiment, these vectors are suitable for the transformation of cells, eukaryotic cells like fungal cells, cells of microorganisms such as yeast or prokaryotic cells. In a particular preferred embodiment, such vectors are suitable for stable transformation of bacterial cells, for example to transcribe the nucleic acid molecule of the present invention. For example, the vector may be pUC18.

The present invention thus relates to a vector such as pUC18 containing a nucleic acid molecule of the present invention coding for a fusion protein as described and provided herein. The present invention therefore relates to a vector such a pUC18 containing a nucleic acid molecule encoding the amino acids 20-217 and 220-446 of SEQ ID NO: 1 fused together, or the sequence SEQ ID NO:1; the amino acids 20-215 and 218-444 of SEQ ID NO: 2 fused together, or the sequence SEQ ID NO: 2; the amino acids 20-217 and 220-446 of SEQ ID NO: 3 fused together, or the sequence SEQ ID NO:3; or the amino acids 20-217 and 220-446 of SEQ ID NO: 4 fused together, or the sequence SEQ ID NO:4.

Particularly, the present invention relates to a vector such as pUC18 containing a nucleic acid molecule comprising the nucleotide sequence 73-666 of SEQ ID NO : 5, or 73-660 of SEQ ID NO : 6, or 73-666 of SEQ ID NO : 7 or 73-666 of SEQ ID NO : 8. Preferably, these vectors also comprise the nucleotide sequence of SEQ ID NO : 9, particularly nucleotides 7-693. Generally, the vector may be capable of expressing said nucleic acid molecule in a eukaryotic host cell.

In one aspect of the invention, the vector as provided is an expression vector. Generally, expression vectors have been widely described in the literature. The expression vector may contain a selection marker gene and a replication-origin ensuring replication in the host. The expression vector may comprise a promoter. He may further comprise a termination signal for transcription. Between, the promoter and the termination signal there is preferably at least one restriction site or a polylinker which enables the insertion of a nucleic acid sequence/molecule desired to be expressed. In an embodiment, the vector is capable of expressing the protein *in vivo,* say the vector, once administered to a patient, is capable of expressing the protein *in situ.*

It is be understood that when the vector provided herein is generated by taking advantage of an expression vector known in the prior art that already comprises a promoter suitable to be employed in context of this invention, for example expression of a UNC5-fusion protein as described herein, the nucleic acid molecule is inserted into that vector in a manner that the resulting vector comprises preferably only one promoter suitable to be employed in context of this invention. The promoter may generally be heterologous or homologous. The vector described herein may also encompass more than one promoter, each respective promoter may be heterologous or homologous. The skilled person knows how such insertion can be put into practice. For example, the promoter can be excised either from the nucleic acid construct or from the expression vector prior to ligation.

The proteins according to the invention are preferably produced by recombinant means. Preferably, the protein expression is in eukaryotic cell with subsequent isolation of the polypeptide and usually purification to a pharmaceutically acceptable purity. For the protein expression, nucleic acids encoding the protein thereof are inserted into expression vectors by standard methods. Expression is performed in appropriate stable eukaryotic host cells like CHO cells, NS0 cells, SP2/0 cells, HEK293 cells, COS cells, and the protein is recovered from the cells (supernatant or cells after lysis). HEK293 cells appeared to be very suitable for this aim and forms a particular embodiment.

In an embodiment, the nucleic acid molecule of the present invention and/or the vector into which the polynucleotide described herein is cloned may be transduced, transformed or transfected or otherwise introduced into a host cell. For example, the host cell is a eukaryotic or a prokaryotic cell, preferably a eukaryotic cell. As a non-limiting example, the host cell is a mammalian cell. The host cell described herein is intended to be particularly useful for generating the UNC5-fusion proteins described and provided in the present invention.

Generally, the host cell described hereinabove may be a prokaryotic or eukaryotic cell, preferably a eukaryotic cell, comprising a nucleic acid molecule provided in the present invention or the vector described herein or a cell derived from such a cell and containing the nucleic acid molecule or the vector described herein. In a preferred embodiment, the host cell comprises, *i.e.* is genetically modified with the nucleic acid molecule of the present invention or the vector described herein in such a way that it contains the nucleic acid molecule of the present invention integrated into the genome. For example, such host cell described herein may be a human, yeast, or fungus cell. In one particular aspect, the host cell is capable to transcribe the nucleic acid molecule of the present invention. An overview of examples of different corresponding expression systems to be used for generating the host cell described herein is for instance contained in Methods in Enzymology 153 (1987), 385-516, in Bitter (Methods in Enzymology 153 (1987), 516-544), in Sawers (Applied Microbiology and Biotechnology 46 (1996), Billman-Jacobe (Current Opinion in Biotechnology 7 (1996), 500-4), Hockney (Trends in Biotechnology 12 (1994), 456-463), and in Griffiths (Methods in Molecular Biology 75 (1997), 427-440). The transformation or genetically engineering of the host cell with a nucleic acid molecule of the present invention or vector described herein can be carried out by standard methods, as for instance described in Sambrook and Russell (2001), Molecular Cloning : A Laboratory Manual, CHS Press, Cold Spring Harbor, NY USA ; Methods in Yeast Genetics, A Laboratory Course Manual, Cold Spring Harbor Laboratory Press, 1990.

In one aspect of the present invention, the host cell comprising the nucleic acid molecule provided herein or a vector described herein may be a HEK293 cell or a HEK293-Freestyle cell (Human embryonic kidney cell line 293, Invitrogen). Accordingly the present invention relates to an HEK293 cell or HEK293-Freestyle cell comprising a nucleic acid molecule encoding the amino acids 20-217 and 220-446 of SEQ ID NO: 1 fused together, or the sequence SEQ ID NO:1; the amino acids 20-215 and 218-444 of SEQ ID NO: 2 fused together, or the sequence SEQ ID NO: 2; the amino acids 20-217 and 220-446 of SEQ ID NO: 3 fused together, or the sequence SEQ ID NO:3; or the amino acids 20-217 and 220-446 of SEQ ID NO: 4 fused together, or the sequence SEQ ID NO:4. The present invention also relates to an HEK293 or HEK293-Freestyle cell comprising a nucleic acid molecule comprising the nucleotide sequence 73-666 of SEQ ID NO : 5, or 73-660 of SEQ ID NO : 6, or 73-666 of SEQ ID NO : 7, or 73-666 of SEQ ID NO : 8. Preferably, these vectors also comprise the nucleotide sequence of SEQ ID NO : 9, particularly nucleotides 7-693.

The present invention relates to a method for producing the UNC5-fusion proteins as provided and described herein. This method comprises the steps of expressing a nucleic acid molecule as provided and described herein in a suitable host cell, especially as described herein, and recovering the UNC5-fusion protein from said cell or the cell culture supernatant.

Accordingly, the present invention relates to a method for producing a UNC5-fusion protein comprising or consisting of the amino acids 20-217 and 220-446 of SEQ ID NO: 1 fused together, or the sequence SEQ ID NO:1, preferably amino acids 20-446 of SEQ ID NO:1; the amino acids 20-215 and 218-444 of SEQ ID NO: 2 fused together, or the sequence SEQ ID NO: 2, preferably amino acids 20-444 of SEQ ID NO:2; the amino acids 20-217 and 220-446 of SEQ ID NO: 3 fused together, or the sequence SEQ ID NO:3, preferably amino acids 20-446 of SEQ ID NO:3; or the amino acids 20-217 and 220-446 of SEQ ID NO: 4 fused together, or the sequence SEQ ID NO:4, preferably amino acids 20-446 of SEQ ID NO:4; comprising the steps of expressing a nucleic acid molecule encoding this amino acid sequence in a host cell (e.g., HEK293 cell or HEK293-Freestyle cell) and recovering the UNC5-fusion protein from said cell or the cell supernatant. The present invention relates to a UNC5-fusion protein obtained or obtainable by the method provided and described herein.

The present invention relates to compositions comprising a UNC5-fusion protein as provided herein, a nucleic acid molecule as provided herein, a vector as described herein, and/or a host cell as described herein. These compositions may further comprise a pharmaceutically acceptable carrier, excipient and/or diluent. These compositions may be used as a pharmaceutical and form a pharmaceutical composition or a medicament.

Examples of suitable pharmaceutical carriers are well known in the art. They include phosphate buffered saline solutions, water, emulsions, such as oil/water emulsions, various types of wetting agents, sterile solutions etc. Pharmaceutical compositions comprising such carriers can be formulated by well known conventional methods.

These pharmaceutical compositions can be administered to a subject at a suitable dose, *i.e.* at least 1 mg/kg body weight, e.g. about 10 mg/kg body weight to about 100 mg/kg weight of the subject in which cancer, is to be treated. Administration of the composition may be effected or administered by different ways, *e.g.* orally, (e.g. pill, tablet, buccal, sublingual, disintegrating, capsule, thin film, liquid solution or suspension, powder, solid crystals or liquid), rectally (e.g. suppository, enema) via injection (e.g. intravenously, subcutaneously, intramuscularly, intraperitoneally, intradermally) via inhalation (e.g., intrabronchially), topically, vaginally, epicutaneously or intranasally). The dosage regimen will be determined by the attending physician and clinical factors. As it well known in the medical arts, dosages for any one of patient depends upon many factors, including the patient's size, body surface area, age, the particular compound to be administered, sex time and route of administration, general health, and other drugs being administered concurrently.

The compositions and pharmaceutical compositions of the invention may be administered locally or systemically. Administration will preferably be intravenously or subcutaneously. The compositions and pharmaceutical compositions may also be administered directly to the target site, *e.g.* by biolistic delivery to an internal or external target site or by catheter to a site in an artery.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based in Ringer's dextrose), and the like. Preservatives and others additives may also be present such as, for example, antimicrobials, anti-oxidants, chelating agents, and inert gases and the like. Furthermore, also doses below or above of the exemplary ranges described hereinabove are envisioned, especially considering the aforementioned factors.

As already mentioned, the present invention relates to pharmaceutical compositions comprising a UNC5-fusion protein as provided herein, a nucleic acid molecule as provided herein, a vector as described herein, and/or a host cell as described herein for use in treating cancer. As already mentioned, the pharmaceutical composition may further comprise a pharmaceutically acceptable carrier, excipient and/or diluent as described hereinabove.

The present invention therefore relates to a pharmaceutical composition comprising a UNC5-fusion protein comprising or consisting of the amino acids 20-217 and 220-446 of SEQ ID NO: 1 fused together, or the sequence SEQ ID NO:1, preferably amino acids 20-446 of SEQ ID NO:1; the amino acids 20-215 and 218-444 of SEQ ID NO: 2 fused together, or the sequence SEQ ID NO: 2, preferably amino acids 20-444 of SEQ ID NO:2; the amino acids 20-217 and 220-446 of SEQ ID NO: 3 fused together, or the sequence SEQ ID NO:3, preferably amino acids 20-446 of SEQ ID NO:3; or the amino acids 20-217 and 220-446 of SEQ ID NO: 4 fused together, or the sequence SEQ ID NO:4, preferably amino acids 20-446 of SEQ ID NO:4; and a pharmaceutically acceptable carrier, excipient and/or diluent as described hereinabove, in particular for use in treating cancer.

The present invention therefore relates to a pharmaceutical composition comprising a UNC5A-fusion protein and a UNC5B-fusion protein; or a UNC5A-fusion protein and a UNC5C-fusion protein; or a UNC5A-fusion protein and a UNC5D-fusion protein; or a UNC5B-fusion protein and a UNC5C-fusion protein; or a UNC5B-fusion protein and a UNC5D-fusion protein; or a UNC5C-fusion protein and a UNC5D-fusion protein; or a UNC5A-fusion protein, a UNC5B-fusion protein, a UNC5C-fusion protein, and a UNC5D-fusion protein; and a pharmaceutically acceptable carrier, excipient and/or diluent as described hereinabove, in particular for use in treating cancer. Therefore, the present invention relates to a pharmaceutical composition comprising one or several, especially two, three, or four UNC5-fusion proteins and a pharmaceutically acceptable carrier, excipient and/or diluent as described hereinabove, in particular for use in treating cancer. These fusion proteins may comprise amino acids 20 to 217 of SEQ ID NO: 1, amino acids 20 to 215 of SEQ ID NO: 2, amino acids 20 to 217 of SEQ ID NO: 3, or amino acids 20 to 217 of SEQ ID NO: 4. They may further comprise amino acids 220-446 of SEQ ID NO:1. These fusion proteins may comprise amino acid sequences selected from SEQ ID NO : 1, preferably amino acids 20-446 of SEQ ID NO:1, SEQ ID NO : 2, preferably amino acids 20-444 of SEQ ID NO:2, SEQ ID NO : 3, preferably amino acids 20-446 of SEQ ID NO:3, and SEQ ID NO : 4, preferably amino acids 20-446 of SEQ ID NO:4.

The present invention also relates to a pharmaceutical composition comprising a nucleic acid molecule encoding a fusion protein according to the invention, or a vector according to the invention, and a pharmaceutically acceptable carrier, excipient and/or diluent as described hereinabove, in particular for use in treating cancer. The composition may comprise several nucleic acid molecules encoding a UNC5A-fusion protein and a UNC5B-fusion protein; or a UNC5A-fusion protein and a UNC5C-fusion protein; or a UNC5A-fusion protein and a UNC5D-fusion protein; or a UNC5B-fusion protein and a UNC5C-fusion protein; or a UNC5B-fusion protein and a UNC5D-fusion protein; or a UNC5C-fusion protein and a UNC5D-fusion protein; or a UNC5A-fusion protein, a UNC5B-fusion protein, a UNC5C-fusion protein, and a UNC5D-fusion protein; and a pharmaceutically acceptable carrier, excipient and/or diluent as described hereinabove, in particular for use in treating cancer. Therefore, the present invention relates to a pharmaceutical composition comprising one or several, especially two, three, or four nucleic acid molecules encoding one or several, especially two, three, or four UNC5-fusion proteins, or the corresponding expression vectors, and a pharmaceutically acceptable carrier, excipient and/or diluent as described hereinabove, in particular for use in treating cancer. These fusion proteins comprise amino acids 20 to 217 of SEQ ID NO: 1, amino acids 20 to 215 of SEQ ID NO: 2, amino acids 20 to 217 of SEQ ID NO: 3, or amino acids 20 to 217 of SEQ ID NO: 4. They may further comprise amino acids 220-446 of SEQ ID NO:1. These fusion proteins may comprise amino acid sequences selected from SEQ ID NO : 1, preferably amino acids 20-446 of SEQ ID NO:1, SEQ ID NO : 2, preferably amino acids 20-444 of SEQ ID NO:2, SEQ ID NO : 3, preferably amino acids 20-446 of SEQ ID NO:3, and SEQ ID NO : 4, preferably amino acids 20-446 of SEQ ID NO:4.

The present invention thus also relates to a pharmaceutical composition comprising a nucleic acid molecule encoding an UNC5 fusion protein comprising amino acids 20 to 217 of SEQ ID NO: 1, amino acids 20 to 215 of SEQ ID NO: 2, amino acids 20 to 217 of SEQ ID NO: 3, or amino acids 20 to 217 of SEQ ID NO: 4 and a pharmaceutically acceptable carrier, excipient and/or diluent as described hereinabove, in particular for use in treating cancer. It also relates to a pharmaceutical composition comprising a nucleic acid molecule encoding an UNC5 fusion protein comprising the amino acids 20-217 and 220-446 of SEQ ID NO: 1 fused together, or the sequence SEQ ID NO:1; the amino acids 20-215 and 218-444 of SEQ ID NO: 2 fused together, or the sequence SEQ ID NO: 2; the amino acids 20-217 and 220-446 of SEQ ID NO: 3 fused together, or the sequence SEQ ID NO:3; or the amino acids 20-217 and 220-446 of SEQ ID NO: 4 fused together, or the sequence SEQ ID NO:4; and a pharmaceutically acceptable carrier, excipient and/or diluent as described hereinabove, in particular for use in treating cancer.

The present invention relates to a pharmaceutical composition comprising a nucleic acid molecule comprising the nucleotide sequence 73-666 of SEQ ID NO : 5, or 73-660 of SEQ ID NO : 6, or 73-666 of SEQ ID NO : 7, or 73-666 of SEQ ID NO : 8 (preferably, these vectors also comprise the nucleotide sequence of SEQ ID NO : 9, particularly nucleotides 7-693) and a pharmaceutically acceptable carrier, excipient and/or diluent as described hereinabove, in particular for use in treating cancer.

The present invention relates to a pharmaceutical composition comprising a vector as described herein containing a nucleic acid molecule comprising the nucleotide sequence 73-666 of SEQ ID NO : 5, or 73-660 of SEQ ID NO : 6, or 73-666 of SEQ ID NO : 7 , or 73-666 of SEQ ID NO : 8 (preferably, these vectors also comprise the nucleotide sequence of SEQ ID NO : 9, particularly nucleotides 7-693) and a pharmaceutically acceptable carrier, excipient and/or diluent as described hereinabove, in particular for use in treating cancer.

Preferably, the nucleic acids and vectors according to the invention allow the production of a mature fusion protein lacking the signal peptide.

The present invention relates to a pharmaceutical composition comprising a host cell as described herein containing a nucleic acid molecule or a vector as provided and described herein and a pharmaceutically acceptable carrier, excipient and/or diluent as described hereinabove, in particular for use in treating cancer.

The present invention further relates to the use of a UNC5-fusion protein as provided herein, a nucleic acid molecule as provided herein, a vector or a host cell as described herein, for the manufacture of a medicament for treating cancer.

The present invention also relates to a method of treating cancer, in a subject by administering a UNC5-fusion protein as provided herein, a nucleic acid molecule as provided herein, a vector or a host cell as described herein to the subject in need thereof. A sufficient amount and/or a suitable dosage regimen is used as specified herein.

The invention will now be described with further details by reference with the following figures.

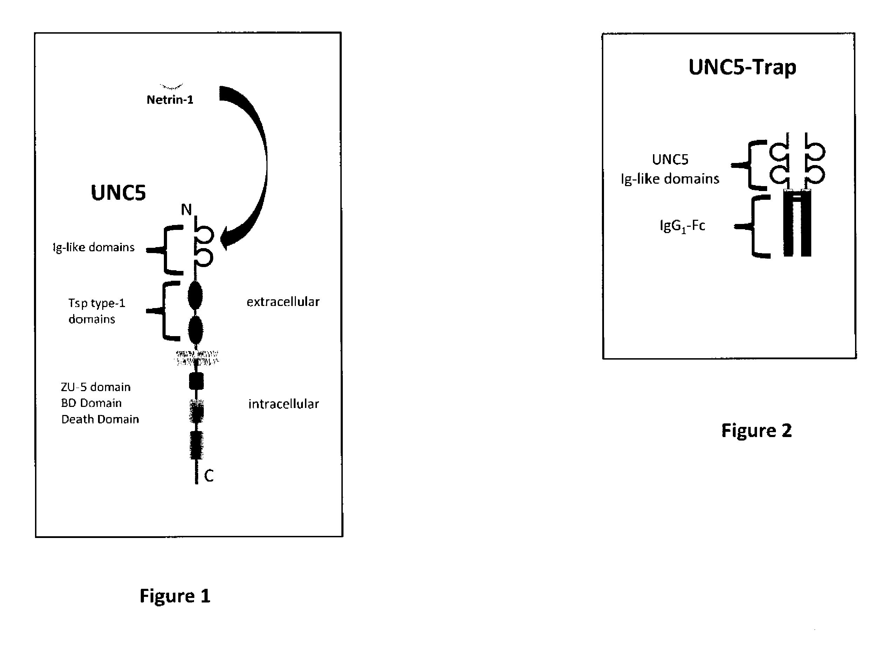

**Figure 1****:** Domain architecture of a UNC5 (UNC5A, B, C or D) receptor and the binding of the ligand netrin-1. lg-like: immunoglobulin-like domains, Tsp-type 1: Thrombospondin type 1 domains. ZU-5, Domain present in ZO-1 and Unc5-like netrin receptors, BD domain: domain binding DCC.
**Figure 2****:** Schematic presentation of the domain architecture of UNC5-TRAP (UNC5A, B, C or D - TRAP) fusion protein as described in the present invention.

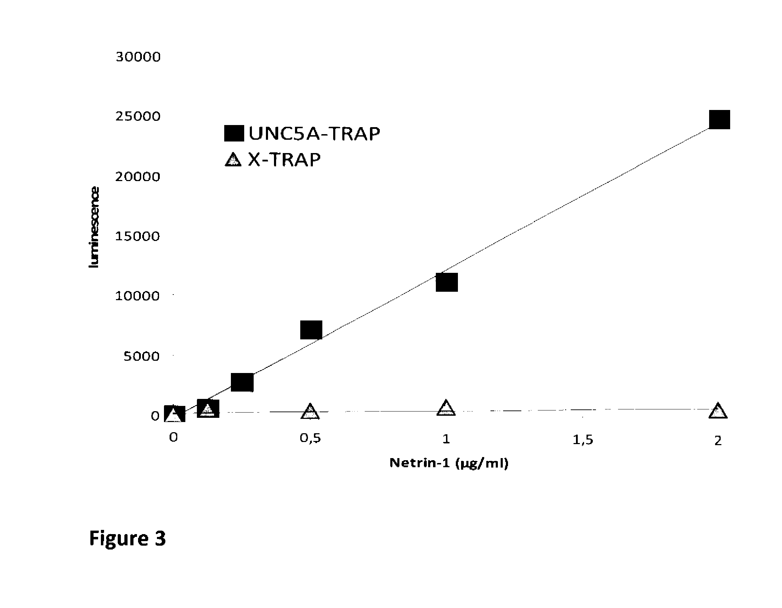

**Figure 3****:** ELISA binding assay of Netrin-1 to UNC5A-TRAP. Various concentrations of FLAG-tagged Netrin-1 (APOTECH) were incubated onto 96-well microtiter plate coated with UNC5A-TRAP or with an unrelated recombinant Fc chimera. Bound Netrin-1 was detected using an anti-Flag antibody conjugated with horseradish peroxidase (SIGMA) and a chemiluminescent substrate (PIERCE ECL western blotting substrate). The luminescent was read on a Tecan Infinite F-500 luminometer.

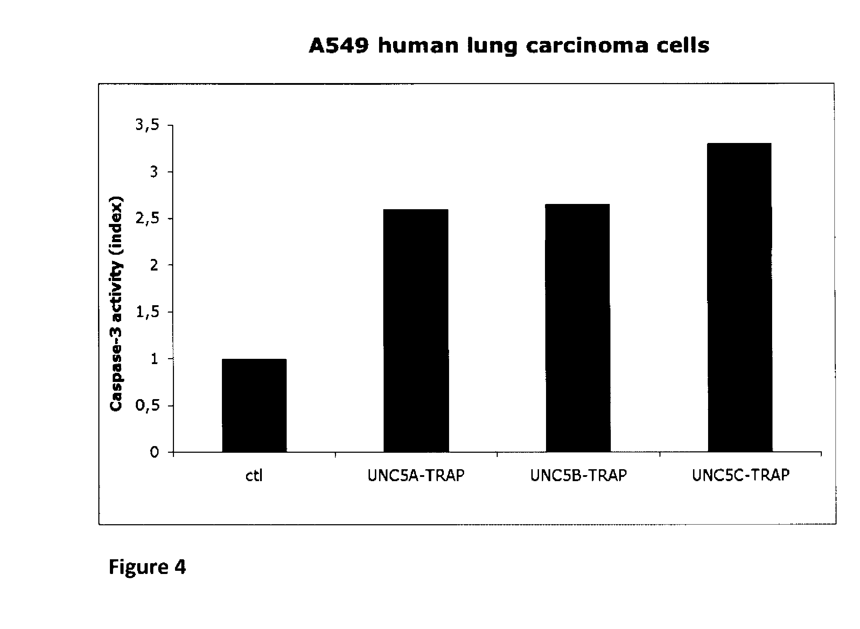

**Figure 4****:** Caspase-3 activation assay in A549 cells using different UNC5-TRAP : UNC5A-TRAP, UNC5B-TRAP, UNC5C-TRAP. Caspase-3 activities in lysates of lung cancer cells A549 treated with UNC5A-TRAP, UNC5B-TRAP or UNC5C-TRAP are graphed as relative units normalized to buffer-treated control cells (ctl).

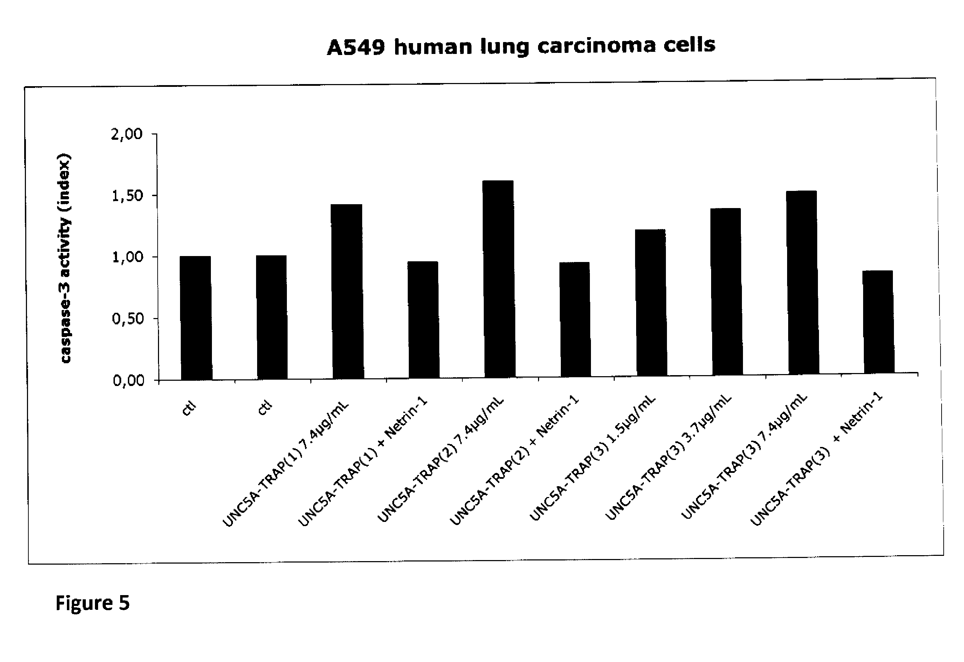

**Figure 5****:** Caspase-3 activation assays using UNC5A-TRAP in Lung A549 cells. Caspase-3 activity in lysates of cells A549 are graphed as relative units normalized to buffer-treated controls cells (ctl). Different batches of UNC5A-TRAP (noted UNC5A-TRAP (1), UNC5A-TRAP(2) and UNC5A-TRAP (3) and different concentrations of UNC5A-TRAP were assayed. The assays were performed in presence or absence of recombinant netrin-1 (150 ng/mL) to challenge a reversion of caspase-3 activation of the UNC5A-TRAP by netrin-1.

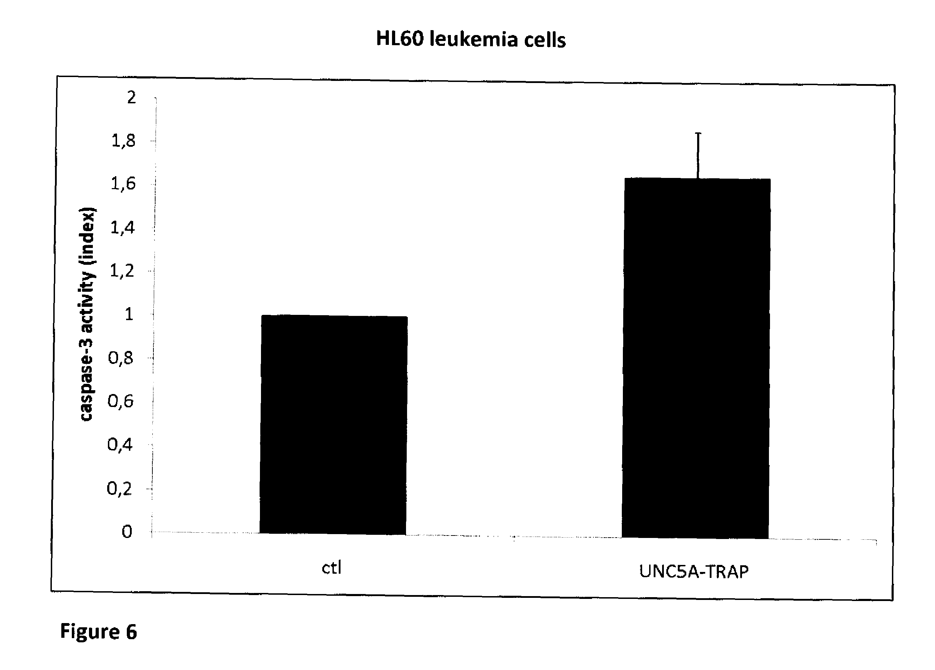

**Figure 6****:** Caspase-3 activation assays using UNC5A-TRAP in HL60 Human promyelocytic leukemia cells. Caspase-3 activity in lysates of HL60 cells are graphed as relative units normalized to buffer-treated controls cells (ctl). Addition of an excess of recombinant netrin-1 (150 ng/mL) is used to possibly reverse caspase-3 activation of the UNC5A-TRAP. 15 µg/mL of UNC5A-TRAP is used in HL60.

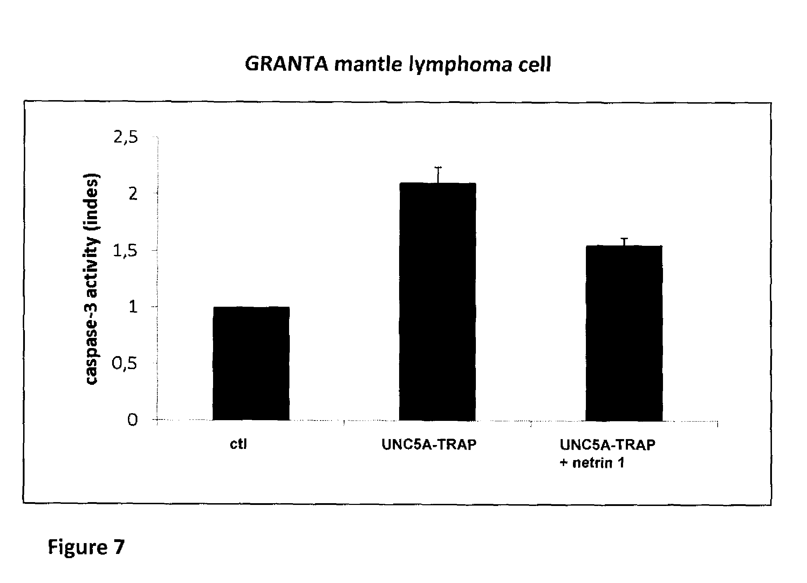

**Figure 7****:** Caspase-3 activation assays using UNC5A-TRAP in mantle lymphoma GRANTA cells. Caspase-3 activity in lysates of GRANTA cells are graphed as relative units normalized to buffer-treated controls cells (ctl). 20 µg/mL of UNC5A-TRAP is used in GRANTA cells and addition of an excess of recombinant netrin-1 (150 ng/mL) is used to possibly reverse caspase-3 activation of the UNC5A-TRAP .

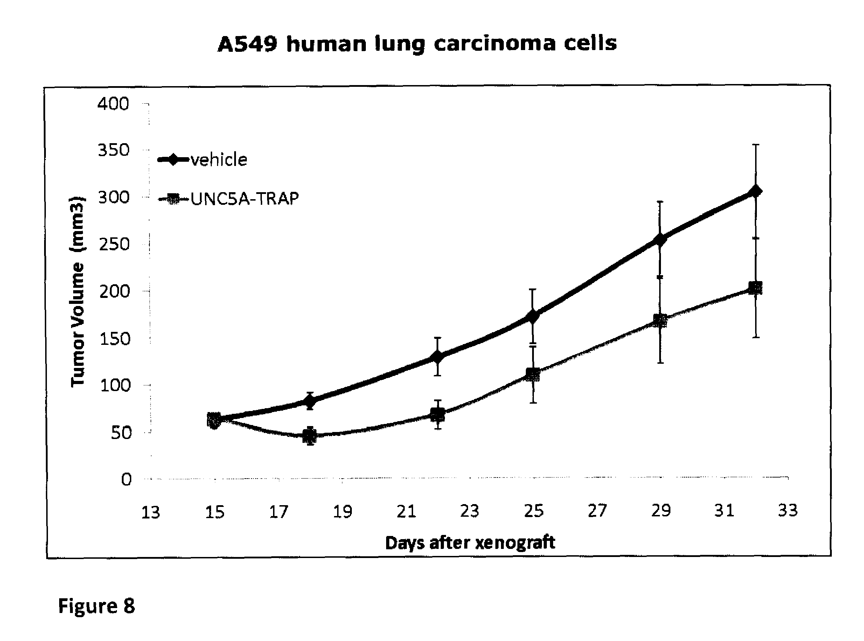

**Figure 8****:** Growth inhibition of A549 human lung carcinoma xenografts in mice treated with UNC5A-TRAP. Tumor growth of subcutaneous xenografts of A549 lung cancer cells in nude mice. Tumor volume in mm³ (y-axis) is determined by calliper measurements. Intraperitoneal injection (i.p.) of the UNC5A-TRAP (10 mg/kg) is performed twice a week. Treatment started at day 15 post-xenograft.The vehicle is injected as a control.

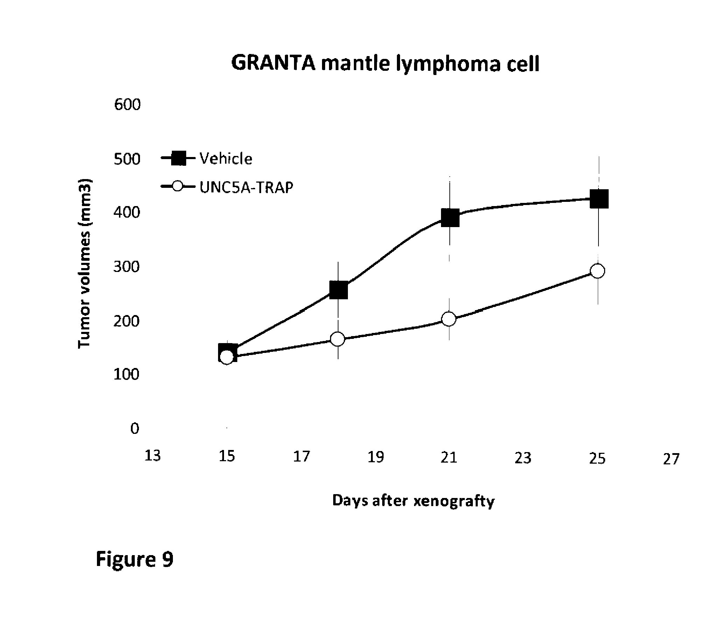

**Figure 9****:** Growth inhibition of GRANTA human mantle lymphoma xenografts in mice treated with UNC5A-TRAP. Tumor growth of subcutaneous xenografts of human mantle lymphoma GRANTA cells in nude mice. Tumor volume in mm³ (y-axis) is determined by calliper measurements. Intraperitoneal injection (i.p.) of the UNC5A-TRAP (20 mg/kg) is performed 5 times a week. Treatment started at day 15 post-xenograft.. The vehicle is injected as a control.

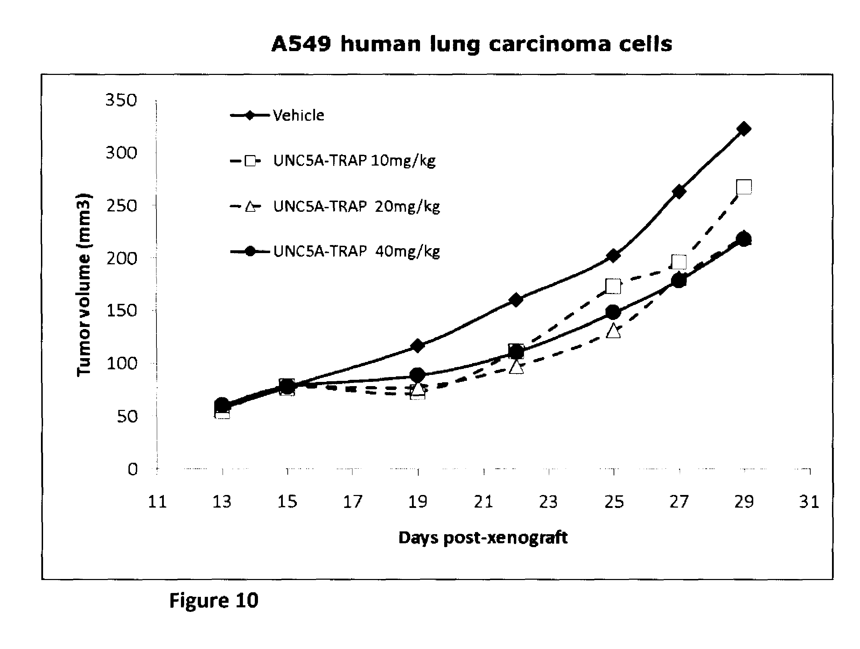

**Figure 10****:** Growth inhibition of A549 xenografts in mice treated with UNC5A-TRAP : dose effect. Xenografts of A549 lung cancer cells were implanted subcutaneously in nude mice. Tumor volume in mm³ (y-axis) was determined by calliper measurements. Treatment started at day 13 post-xenograft. Twice a week, the mice received an Intraperitoneal (i.p.) injection of UNC5A-TRAP at 10, 20 or 40 mg/kg) or the vehicule alone.

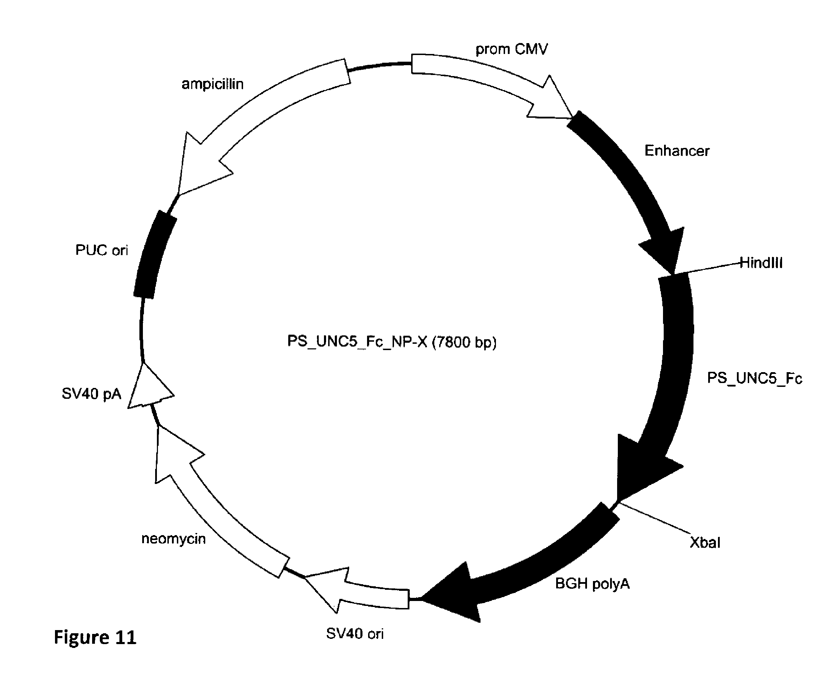

**Figure 11****:** Map of expression plasmid NP-X.

### Sequence listing:

| SEQ ID NO: | Amino acid sequence | Nucleic acid sequence |
|---|---|---|
| 1 | UNC5A-TRAP with peptide signal | |
| 2 | UNC5B-TRAP with peptide signal | |
| 3 | UNC5C-TRAP with peptide signal | |
| 4 | UNC5D-TRAP with peptide signal | |
| 5 | | UNC5A |
| 6 | | UNC5B |
| 7 | | UNC5C |
| 8 | | UNC5D |
| 9 | | Human IgG1 Fc |
| 10 | | primer |
| 11 | | primer |

### Example 1 : UNC5-TRAP fusion proteins

### Plasmid construction

Standard methods were used to manipulate DNA as described in Sambrook, J. et al., Molecular Cloning: A laboratory manual; Cold Spring Harbor Laboratory Press, Cold Spring Harbor, New York, 1989. The molecular biological reagents were used according to the manufacturer's instructions. Desired gene segments were prepared by gene synthesis. The synthesized gene fragments were cloned into a specified expression vector. The DNA sequence of the subcloned gene fragments were confirmed by DNA sequencing.

Expression plasmid is represented on Figure 11 and noted PS-UNC5-Fc-NP-X (PS for peptide signal, and X may be V-01 for UNC5A, V-02 for UNC5B and V-03 for UNC5C.

This vector is an expression plasmid e.g. for transient expression of an artificial lg Fc fusion protein in which the lg-like domains of the human UNC5A, B, or C receptor is fused to the hinge region of human IgG 1 antibody (Fc constant region; Hinge-CH2-CH3) with the introduction of a 2 amino acid artificial linker sequence.

Chemical gene synthesis was used to prepare the DNA segments of 672 (SEQ ID NO: 5, 7) or 676 bps (SEQ ID NO: 6) flanked by a unique Hindlll and Kpnl restriction endonuclease at the 5'- and the 3'-end, respectively. Similarly, was prepared the DNA segment of 699 bps (SEQ ID N0: 9) flanked by a unique Kpnl and Xbal restriction endonuclease at the 5'- and the 3'-end, respectively. A DNA segment coding for the open reading frame (ORF) of the desired UNC5-fusion protein (UNC5-TRAP fusion protein) (SEQ ID NO: 1, 2, 3) with the Kappa 2 signal peptide at the N-terminal position was obtained by ligation of the two DNA segments cited above. The gene was introduced in an expression vector (NP-V) to the immediate promoter of CMV-IE and enhancer hE1 and the bovine growth hormone (bGH) polyadenylation site.

The UNC5-fusion protein (UNC5-TRAP fusion protein) is composed of a murine signal sequence (amino acids 1 to 19 of SEQ ID NO: 1, 2 or 3), the two immunoglobulin-like domains of the human UNC5 receptor (UNC5A : amino acids 20 to 217 of SEQ ID NO: 1; amino acids 34 to 240 af UNC5A; amino acid sequence: UniProt ID: Q6ZN44; UNC5B : amino acids 20 to 215 of SEQ ID NO: 2; amino acids 49 to 244 of UNC5B; amino acid sequence: UniProt ID: Q8IZJ1; UNC5C : amino acids 20 to 217 of SEQ ID NO: 3; amino acids 61 to 258 of UNC5C; amino acid sequence: UniProt ID: 095185; two amino acid linker (from cloning site; amino acids 199 to 200 of SEQ ID NO: 3) and the human IgGI antibody Fc constant region (amino acids 220 to 446 of SEQ ID NO: 1 or 3; amino acids 218 to 444 of SEQ ID NO: 2). The mature UNC5-TRAP fusion proteins lack the signal peptide.

### Transient transfection, expression and purification

Recombinant proteins were obtained by transient transfection of Freestyle HEK 293 cells (invitrogen) growing in suspension in 293 Freestyle culture medium (Invitrogen) with 8 % CO₂ at 37°C. For transfection 293fectin® reagent (Invitrogen) was used according to manufacturer's instructions. Three days after transfection, supernatants were harvested and clarified by centrifugation (10min at 200g). The Fc-fusion proteins were purified using Protein G Sepharose 4 FF according to the manufacturer's instructions. Elutions were performed in 0.1 M Glycin pH 2.8. Eluates were neutralized in 1 M Tris-Hcl pH 9.0 and dialyzed over night against PBS. Final analytics were performed using polyacrylamide gel electrophoresis in denaturing and non denaturing conditions followed by coomassie blue staining or by western blot analysis after nitrocellulose transfer (using an anti-human IgG (Fc specific)-HRP antibody, Sigma).

Recombinant UNC5A-TRAP was also obtained by transient transfection of Freestyle CHO-S cells (Chine Hamster Ovary, Invitrogen) growing in suspension in a chemically defined, animal-component free, serum-free media with 8 % CO₂ at 31 °C. For transfection FreeStyleTM MAX Reagent (Invitrogen) was used according to manufacturer's instructions. The UNC5A-TRAP fusion protein (SEQ ID NO: 1) could be secreted with high efficiency at a rate of at least 300 mg/L at transient expression in Freestyle CHO-S cells. Supernatant was harvested by centrifugation and sterile filtered (0.2 µm). The concentration of UNC5a-TRAP in the supernatant was determined using the BioRad Experion system. The Fc-fusion protein was subsequently purified via cation exchange chromatography followed by Protein A affinity chromatography (PALL Protein A Ceramic Hyper D) according to manufacturer's instructions with the exception of elution in 0.1 M Glycine HCl pH 3.0. The eluate was neutralized with 1 M Tris-HCl pH 9 and dialyzed overnight against 20 mM Citrate, 134 mM NaCl, pH 6.2. Final analytics were performed with the Bio-Rad Experion system for quantification, size verification and presence of contaminants.

**Table : Results of expression**

| Plasmid # | Characteristic | Sequence | MW kDa | Expression yield µg/mL | Expression yield µg/mL |
|---|---|---|---|---|---|
| | | | | (Freestyle HEK 293 supernatant day 3) | (Freestyle CHO-S supernatant day 8) |
| NP-V-01 | UNC5A-Fc | SEQ ID NO. 1 | 48,17 | 3.5 | 300 |
| NP-V-02 | UNC5B-Fc | SEQ ID NO. 2 | 48,31 | 25 | - |
| NP-V-03 | UNC5C-Fc | SEQ ID NO. 3 | 48,55 | 5 | - |

### Example 2 : ELISA-type Binding assay of Netrin-1 to UNC5A-TRAP (figure 3)

White 96-well microtiter plate (Costar 3912 Corning) were incubated overnight at 4°C with 125ng of UNC5A-TRAP or of an unrelated recombinant Fc chimera in 100µL of Phosphate buffer saline (PBS). After two washings with 300µL of PBS-0.05% Tween-20 (PBST), 100µL of PBST-5%BSA containing or not 10 to 200 ng FLAG-tagged Netrin-1 (Apotech) was added and the plate was incubated 1 hour at room temperature. After four washings with 300µL of PBS-T, 100µL of a monoclonal Anti-FLAG M2 antibody conjugated to horse radish peroxidase (HRP) (Sigma A8592) diluted in PBST-3% BSA was added and the plate was incubated 1 hour at room temperature. After four washings with 300µL of PBS-T, 100µL of a luminescent substrate of HRP (ECL western blotting substrate, PIERCE) was added. After 5 to 10 minutes, the luminescence is read on a Tecan Infinite F-500 luminometer.

### .Example 3 : caspase-3 activation assay with A549 cells treated with UNC5A-, UNC5B- or UNC5C-TRAP (Figure 4)

Adherent A549 human lung carcinoma cells were grown in DMEM medium supplemented with 10% foetal calf serum. On day 0, A549 cells (collected from culture flasks with cell density below 6 × 10⁵ cells/cm²) were plated in serum free DMEM medium (1.8 x 10⁵ cells per well in six-well plates with 1 mL per well). On day 1, the medium was replaced with 1 mL fresh serum-free DMEM medium containing 1.5 µg/mL of UNC5A-, UNC5B-, UNC5C-TRAP. Treatments were done on two wells per condition. On day 2, floating and adherent cells from the 2 identically treated wells were harvested and pooled. Cell pellets were resuspended in 55 µL of lysis buffer provided in the Caspase 3/CPP32 Fluorimetric Assay Kit (Gentaur Biovision, Brussels, Belgium). Apoptosis was then monitored by measuring caspase-3 activity using Caspase 3/CPP32 Fluorimetric Assay Kit (Gentaur Biovision, Brussels, Belgium). All values were normalized to the vehicle control sample.

### Example 4 : caspase-3 activation assay with A549 cells treated with UNC5A-TRAP (Figure 5)

A549 cells were cultured and plated as described in Example 3. On day 1, the medium was replaced with 1 mL fresh serum-free DMEM medium containing either vehicle or different amount of UNC5A-TRAP in presence or absence of 150 ng/mL netrin-1. On day 2, the cells were processed and caspase assays were performed as described in Example 3.

### Example 5 : caspase-3 activation assay with HL60 cells cells treated with UNC5A-TRAP (Figure 6)

Non adherent HL60 human leukemia cells were grown in RPMI medium containing 10% foetal calf serum. On day 0, HL60cells (collected from culture with a minimal density of 1 x 10⁶ cells/ml) were plated in serum free low glucose (1 g/L) RPMI medium (2 x 10⁵ cells per well in six-well plates with 1 mL per well). Treatment started at the time of seeding by the addition of 15 µg/mL UNC5A-TRAP (or vehicle alone). Treatments were done on four wells per condition. On day 1, the cells from the 4 identically treated wells were harvested and pooled, and caspase assay was performed as described in Example 3.

### Example 6 : caspase-3 activation assay with GRANTA cells treated with UNC5A-TRAP (Figure 7)

Non adherent GRANTA human mantle lymphoma cells were grown in DMEM medium supplemented with 10% foetal calf serum. On day 0, cells (collected from culture with a minimal density of 2 x 10⁶ cells/ml) were plated in serum free low glucose (1 g/L) DMEM medium (5 x 10⁵ cells per well in six-well plates with 1 mL per well). Treatment started at the time of seeding by the addition of 15 µg/mL UNC5A-TRAP (or vehicle alone) in presence or absence of 150 ng/mL Netrin-1 (AXORA). Treatments were done on two wells per condition. On day 3, the cells from the 2 identically treated wells were harvested and pooled, and caspase assay was performed as described in Example 3.

### Example 7 : in vivo tumor growth inhibition of A549 human lung carcinoma cells xenografts. (Figures 8 and 9)

Seven-week-old (20-22 g body weight) female athymic nu/nu mice were obtained from Charles River animal facility. The mice were housed in sterilized filter-topped cages and maintained in a pathogen-free animal facility. A549 cells (grown in culture flask in DMEM medium supplemented with 10% foetal calf serum and collected from culture flasks with cell density below 6 x 10⁵ cells/cm²) were implanted by sub-cutaneous (s.c.) injection of 10⁷ cells in 200µL of PBS into the right flank of the mice. When tumors were established (V≈100mm³, approximately 15 days post-injection), mice were treated with UNC5A-TRAP. Tumor sizes were measured with a caliper. The tumor volume was calculated with the formula v = 0.5*(length*width²) (n=10 mice per group).

A set of xenografted mice received intra-peritoneal (i.p.) injection twice a week of UNC5A-TRAP at 10 mg/Kg. Control animals received injection of the vehicule (Figure 8)

Another set of animals received intra-peritoneal (i.p.) injection twice a week of UNC5A-TRAP at 10, 20 or 40 mg/Kg. Control animals received injection of the vehicule. (Figure 10)

### Example 8 : in vivo tumor growth inhibition of GRANTA human mantle lymphoma cells xenografts (Figure 9)

Female athymic nu/nu mice were purchased and hosted as described in example 7. GRANTA cells (grown in DMEM medium supplemented with 10% foetal calf serum and collected from culture with a minimal density of 2 x 10⁶ cells/ml) were implanted by s.c. injection of 10⁶ cells in 200µL of PBS into the right flank of the mice. When tumors were established (V≈100mm³, approximately 15 days post-injection), mice were treated five times per week with i.p. injection of UNC5A-TRAP at 20 mg/Kg. Tumor sizes were measured with a caliper. The tumor volume was calculated with the formula v = 0.5*(length*width²) (n=10 mice).

### Example 9. Quantitative RT-PCR for assessing overexpression of netrin-1:

Total RNA is extracted using NucleoSpin^{Ⓡ} RNA II Kit (Macherey Nagel, Düren, Germany) according to manufacturer's protocol. RT-PCR reactions are performed with iScript^{®} cDNA Synthesis Kit (Biorad). One 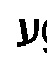 g total RNA is reverse-transcribed using the following program: 25°C for 5 min, 42°C for 30 min and 85°C for 5 min. For expression studies, the target transcripts are amplified in LightCycler^{®} 2.0 apparatus (Roche Applied Science), using the LightCycler FastStart DNA Master SYBR Green I Kit (Roche Applied Science). Expression of target genes is normalized to glyceraldehyde 3-phosphate dehydrogenase (GAPDH) and phosphoglycerate kinase (PGK) genes, used as housekeeping genes. The amount of target transcripts, normalized to the housekeeping gene, is calculated using the comparative C_{T} method. A validation experiment is performed, in order to demonstrate that efficiencies of target and housekeeping genes are approximately equal. The sequences of the primers are as follows:
Forward primer: aaaagtactgcaagaaggactatgc SEQ ID NO:10.
Reverse primer: ccctgcttatacacggagatg SEQ ID NO:11.

### Example 10: Netrin-1 protein quantification in human cancer cells:

For immunoblot analysis, cells are lysed by sonication in modified RIPA buffer (50mM Tris-HCl, pH7.5, 150mM NaCl, 1% NP-40, 0.5% sodium deoxycholate, 0.1% SDS, 1 mM EDTA, protease inhibitor cocktail and 5mM DTT) and incubated 1h at 4°C. Cellular debris are pelletted by centrifugation (10.000g 15' at 4°C) and protein extracts (200 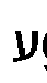 g per lane) are loaded onto 10% SDS-polyacrylamide gels and blotted onto PVDF sheets (Millipore Corporation, Billerica, MA, U.S.A.). Filters are blocked with 10% non-fat dried milk and 5% BSA in PBS/0.1 % Tween 20 (PBS-T) over-night and then incubated for 2h with rabbit polyclonal α-netrin-1 (dilution 1:500, clone H104, Santa Cruz Biotechnology, Santa Cruz, CA, USA) and mouse monoclonal β-actin (Santa Cruz Biotechnologies) antibodies. After three washes with PBS-T, filters are incubated with the appropriate HRP-conjugated secondary antibody (1:10000, Jackson ImmunoResearch, Suffolk, UK) for 1 h. Detection is performed using West Dura Chemiluminescence System (Pierce, Rockford, IL, U.S.A.).

For immunofluorescence study, cells are detached, centrifuged on cover slips with a cytospiner (Shandon Cytospin 3, Thermo Scientific) and fixed for 30 minutes with 4% (v/v) paraformaldehyde. Cells are then permeabilized for 30 minutes in 0.2% Triton X-100/PBS and blocked in PBS containing 2% BSA and 2% normal donkey serum. Endogenous netrin-1 is stained using rat monoclonal α-netrin-1 antibody (R&D systems) and Alexa-488 Donkey anti-rat IgG (Molecular probes). Nuclei are counterstained using Hoescht staining (Sigma).

### Example 11: Example of cancers over-expressing netrin-1 and expressing DCC and/or UNC5A and/or B and/or C and/or D to be candidate for the treatment with a UNC5-TRAP.

The percentage of netrin-1 overexpressing cases is given for each type of cancers for which expression of netrin-1 and its receptors have been quantified.
- 60 % of metastatic breast cancer (Fitamant et al., PNAS 2008),
- 47 % of non-small cell lung cancer (Delloye-Bourgeois et al., JNCI 2009),
- 38 % of aggressive neuroblastoma (Delloye-Bourgeois et al., J. Exp. Med. 2009),
- 61 % of pancreatic adenocarcinoma (Link et al., Annals of Chir. Onco. 2007; Dumartin et al., Gastro 2010),
- 100 % of primary melanoma (n=7), melanoma metastasis (n=6) (Kaufmann et al., Cellular Oncology 2009),
- 76 % of ovarian cancers (Panastasiou et al0., Oncotarget 2011),
- 65% of glioblastoma,
- 60 % of acute myeloid leukemia and chronic lymphocytic leukemia
- 50 % of aggressive B-cell lymphoma,
- 30 % of sarcoma,
- 40 % of renal adenocarcinoma,
- 22 % of head and neck cancers,
- Testicular cancers (36 % of embryonal carcinoma, 50 % of teratoma, 100 % of yolk sac tumors)
- 50 % of kidney cancers,
- 26 % of stomach cancers,
- 19 % of uterus cancers.

## Claims

1. A UNC5-fusion protein comprising the two lg-like domains of UNC5A, UNC5B, UNC5C or UNC5D and an antibody Fc-part.

2. The fusion protein of claim 1, comprising the sequence of amino acids 20 to 217 of SEQ ID NO: 1, or the sequence of amino acids 20 to 215 of SEQ ID NO: 2, or the sequence of amino acids 20 to 217 of SEQ ID NO: 3, or the sequence of amino acids 20 to 217 of SEQ ID NO: 4.

3. The fusion protein of claim 1 or 2, comprising the Fc part of an IgG, preferably IgG1.

4. The fusion protein of claim 3, consisting of the sequence of amino acids 20-446 of SEQ ID NO: 1, or the sequence of amino acids 20-444 of SEQ ID NO: 2, or the sequence of amino acids 20-446 of SEQ ID NO: 3, or the sequence of amino acids 20-444 of SEQ ID NO: 4.

5. The fusion protein of any one of claims 1 to 4, which is single chain or double chain.

6. A nucleic acid molecule encoding the UNC5-fusion protein according to any one of claims 1 to 5.

7. The nucleic acid molecule according to claim 6, which comprises nucleotide sequence 73-666 of SEQ ID NO : 5, or 73-660 of SEQ ID NO : 6, or 73-666 of SEQ ID NO : 7, or 73-666 of SEQ ID NO : 8.

8. A vector containing the nucleic acid molecule according to claim 5 or 7, capable of expressing said nucleic acid molecule in a host cell.

9. A host cell comprising the nucleic acid molecule according to claim 5 or 7 or the vector according to claim 7.

10. A method for producing the UNC5-fusion protein according to any one of claims 1 to 6, comprising the steps of expressing a nucleic acid molecule according to claim 5 or 6 in a eukaryotic host cell and recovering the UNC5-fusion protein from said cell or the cell culture supernatant.

11. A UNC5-fusion protein obtainable by the method of claim 10.

12. A pharmaceutical composition comprising the UNC5-fusion protein according to any one of claims 1 to 6, the nucleic acid molecule according to claim 5 or 6, the vector of claim 7, or the host cell of claim 8, and a pharmaceutically acceptable carrier.

13. The pharmaceutical composition of claim 12 or the UNC5-fusion protein of any one of claims 1-5 and 10, for use in the treatment of cancer.

14. The pharmaceutical composition of claim 13, wherein the cancer is a cancer with netrin-1 overexpression.

15. The pharmaceutical composition of claim 13, wherein the cancer with netrin-1 overexpression is metastatic breast cancer, non-small cell lung cancer, aggressive neuroblastoma, pancreatic adenocarcinoma, primary melanoma (n=7), melanoma metastasis (n=6), ovarian cancer, glioblastoma, acute myeloid leukemia, chronic lymphocytic leukemia, aggressive B-cell lymphoma, sarcoma, renal adenocarcinoma, head and neck cancer, Testicular cancer (e.g. embryonal carcinoma, teratoma, yolk sac tumors), kidney cancer, stomach cancer, or uterus cancer.
